# EUROPEAN PATENT APPLICATION

(11) **EP 4 706 678 A1**
(43) Date of publication of application: **11.03.2026**
(21) Application number: 25199133.7
(22) Date of filing: 29.08.2025
(51) Int. Cl.: A61K 40/11, A61K 40/31, A61K 40/42, C12N 15/88

(54) **METHOD FOR PROLIFERATING CAR-T CELLS, AND LIPID PARTICLES AND KIT FOR USE IN THE METHOD**

(30) Priority: 03.09.2024 JP 2024151517
(71) Applicant: KABUSHIKI KAISHA TOSHIBA, Kawasaki-shi, Kanagawa (JP); SHINSHU UNIVERSITY, Matsumoto City, Nagano, 390-8621 (JP)
(72) Inventor: AKAHOSHI, Eiichi, Kawasaki-shi (JP); ISHIHARA, Mitsuko, Kawasaki-shi (JP); NAKAZAWA, Yozo, Matsumoto City 390-8621 (JP); TANAKA, Miyuki, Matsumoto City 390-8621 (JP); YAGYU, Shigeki, Matsumoto City 390-8621 (JP)
(74) Representative: Marks & Clerk LLP

(57) **Abstract**

According to one arrangement, a lipid particle (10) for producing a CAR antigen-expressing cell derived from a CD209 positive cell is provided. The lipid particle (10) includes a lipid membrane (1) having a lumen, and a nucleic acid (2) containing a CAR antigen gene being contained in the lumen.

## Description

### FIELD

The present disclosure relates to a method for proliferating CAR-T cells, and lipid particles and a kit for use in the method.

### BACKGROUND

CAR-T cell therapy, which uses T cells genetically modified to produce chimeric antigen receptors (CARs) that recognizes tumor cells (CAR-T cells), has attracted attention because of its extremely high therapeutic effect on tumors. In this method, a CAR gene is transferred into T cells collected from a patient to produce CAR-T cells, which are then administered to the patient. The administered CAR-T cells kill tumor cells encountered in the patient's body.

In general, production of CAR-T cells often involves a step of stimulating the CAR-T cells in order to allow them to proliferate more efficiently. The stimulation step is performed, for example, by a method of culturing CAR-T cells together with an active factor of T cells (for example, interleukin or the like) or by a method of co-culturing CAR-T cells with peripheral blood mononuclear cells (PBMCs) that express an antigen of a CAR and a co-stimulating factor as antigen-presenting cells.

Although proliferation efficiency of CAR-T cells is practically sufficient even by the conventional proliferation method including the above-mentioned stimulation step, it is possible to suppress production cost of preparations containing CAR-T cells and to enhance the therapeutic effect thereof by producing CAR-T cells with even higher efficiency. Therefore, a method for proliferating CAR-T cells more efficiently than the conventional method is required.

### BRIEF DESCRIPTION OF THE DRAWINGS

FIG. 1 is a cross-sectional view showing an example of a lipid particle of a first arrangement.
FIG. 2 is a diagram showing an example of a method for producing a CAR antigen-expressing cell of the first arrangement.
FIG. 3 is a flowchart showing an example of a method for proliferating CAR-T cells of a second arrangement.
FIG. 4 is a schematic view showing an example of a method for proliferating CAR-T cells of the second arrangement.
FIG. 5 is a graph showing experimental results of Example 1.
FIG. 6 is a graph showing experimental results of Example 2.
FIG. 7 is a graph showing experimental results of Example 3.

### DETAILED DESCRIPTION

According to one arrangement, a method that proliferate a genetically-modified T cell (CAR-T cell) expressing a chimeric antigen receptor (CAR) is provided. The method comprises preparing a cell population containing CAR-T cells, a CD209 positive cell, and the lipid particle, respectively; producing a CAR antigen-presenting cell by bringing the lipid particle into contact with the CD209 positive cell and transfecting the nucleic acid into the CD209 positive cell and, co-culturing the CAR-T cell and the CAR antigen-presenting cell.

According to one arrangement, a lipid particle for producing a CAR antigen-expressing cell derived from a CD209 positive cell is provided. The lipid particle includes a lipid membrane having a lumen, and a nucleic acid containing a CAR antigen gene being contained in the lumen.

According to one arrangement, a kit for producing a CAR antigen-expressing cell is provided. The kit comprises at least the lipid particle for producing a CAR antigen-expressing cell derived from a CD209 positive cell, which includes a lipid membrane having a lumen and a nucleic acid containing a CAR antigen gene being contained in the lumen, and a substance that improves storage stability of the lipid particle.

Hereinafter, lipid particles, a method for proliferating CAR-T cells, and a kit according to arrangements will be described with reference to the drawings. Each drawing is a schematic view for promoting the arrangement and understanding thereof, and the shape, size, ratio, and the like may be different from actual ones, but these can be appropriately changed in design in consideration of the following description and known techniques.

### [First Arrangement]

### · Lipid particle

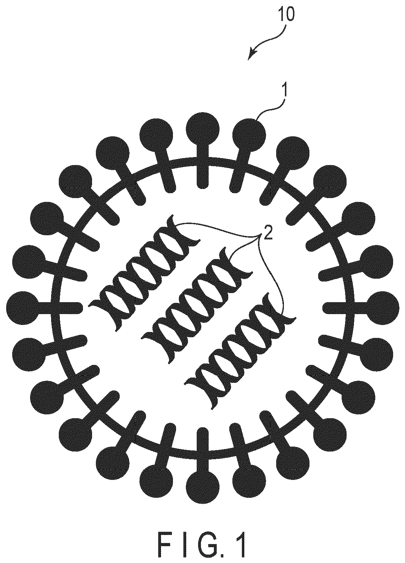

The lipid particle of the first arrangement is used for producing a CAR antigen-expressing cell. As shown in FIG. **1****,** a lipid particle 10 includes, for example, a lipid membrane 1 and a first nucleic acid 2 contained in the lipid membrane 1. The first nucleic acid 2 is a nucleic acid containing a gene encoding a target antigen of a CAR (hereinafter, referred to as "CAR antigenic gene"). More specifically, it is used in a method for producing a CAR antigen-expressing cell by bringing a lipid particle containing a nucleic acid containing a CAR antigen gene into contact with a CD209 positive cell described later and transferring the CAR antigen gene into the CD209 positive cell.

Hereinafter, each component will be described in detail.

### (Lipid membrane)

The lipid particle 10 of the present arrangement is a substantially spherical hollow body including a lipid membrane 1 formed by arranging a plurality of lipid molecules 1a by non-covalent bonds, that is, a liposome. Then, the first nucleic acid 2 is contained in a central lumen 1b. The lipid membrane 1 may be a lipid monomolecular membrane or a lipid bilayer membrane. Further, the lipid membrane 1 may be a single layer of membrane or may be a multilayered membrane.

The material of the lipid membrane 1 may include a base lipid exemplified below, but preferably further contains a lipid compound exemplified below in addition to the base lipid.

As the base lipid, for example, a lipid that is a main component of a biological membrane can be used. The base lipid is a phospholipid or a sphingolipid, such as diacylphosphatidylcholine, diacylphosphatidylethanolamine, ceramide, sphingomyelin, dihydrosphingomyelin, kephalin, or cerebroside, or a combination thereof.

For example, as a base lipid, it is preferable to use
1,2-dioleoyl-sn-glycero-3-phosphoethanolamine (DOPE),
1,2-stearoyl-sn-glycero-3-phosphoethanolamine (DSPE),
1,2-dipalmitoyl-sn-glycero-3-phosphatidylcholine (DPPC),
1-palmitoyl-2-oleoyl-sn-glycero-3-phosphatidylcholine (POPC),
1,2-di-O-octadecyl-3-trimethylammonium propane (DOTMA) ,
1,2-dioleoyl-3-dimethylammonium propane (DODAP),
1,2-dimyristoyl-3-dimethylammonium propane (14:0 DAP),
1,2-dipalmitoyl-3-dimethylammonium propane (16:0 DAP),
1,2-distearoyl-3-dimethylammonium propane (18:0 DAP),
N-(4-carboxybenzyl)-N,N-dimethyl-2,3-bis(oleoyloxy)propane (DOBAQ),
1,2-dioleoyl-3-trimethylammonium propane (DOTAP),
1,2-dioleoyl-sn-glycero-3-phosphochlorin (DOPC),
1,2-dilinoleoyl-sn-glycero-3-phosphochlorin (DLPC),
1,2-dioleoyl-sn-glycero-3-phospho-L-serine (DOPS),
   or
cholesterol or a combination of any of these.

In particular, DOTAP is preferable because it is a cationic lipid, and the acid dissociation constant of the lipid membrane 1 and thus the lipid particle 10 can be adjusted by its content. The base lipid is easily fused with a cell membrane, and in particular, in a case where diacylphosphatidylcholine and diacylphosphatidylethanolamine are used, the structure and particle size of the lipid particle 10 are easily controlled, and the base lipid is easily fused with a cell membrane, which is preferable. A length of a hydrocarbon chain of an acyl group contained in the lipid is preferably C₁₀ to C₂₀. The hydrocarbon chain may be a saturated hydrocarbon group or an unsaturated hydrocarbon group.

The base lipid may be contained in an amount of nearly 100% to the total lipid molecules 1a contained in the lipid membrane 1, but the base lipid of the lipid membrane 1 is preferably contained in an amount of about 30% to about 80% (molar ratio) to the total lipid molecules 1a.

The other lipid compound other than the base lipid is, for example, a biodegradable lipid. Specifically, a biodegradable lipid represented by the formula Q-CHR₂ (hereinafter, referred to as "lipid compound A") can be used.
(wherein,
Q is a nitrogen-containing aliphatic group containing two or more tertiary nitrogen atoms and no oxygen,
Rs are each independently an aliphatic group of C₁₂ to C₂₄,
at least one R includes, in its main chain or side chain, a linking group LR selected from the group consisting of -C(=O)-O-, -O-C(=O)-, -O-C(=O)-O-, -S-C(=O)-, -C(=O)-S-, -C(=O)-NH-, and -NHC(=O)-).

In a case where the lipid membrane 1 contains the lipid compound A, cationic properties of a surface of the lipid particle 10 can be adjusted, thereby reducing obstacles in cell transfection and increasing a transfection rate of nucleic acid.

As the lipid compound A, for example, it is preferable to use lipids having structures represented by the following formulas (1-01) and (1-02) because transfection efficiency of nucleic acid is more excellent. Note that, in the following description, the lipid of formula (1-01) is referred to as FFT10, and the lipid of formula (1-02) is referred to as FFT20.

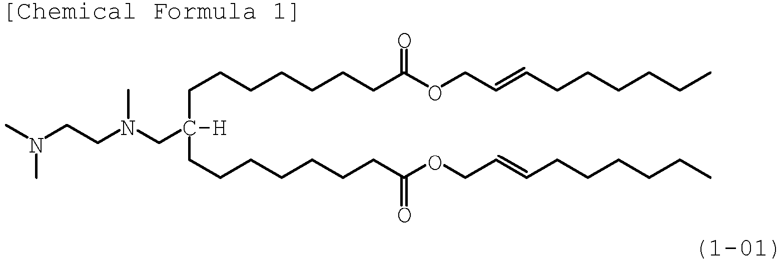

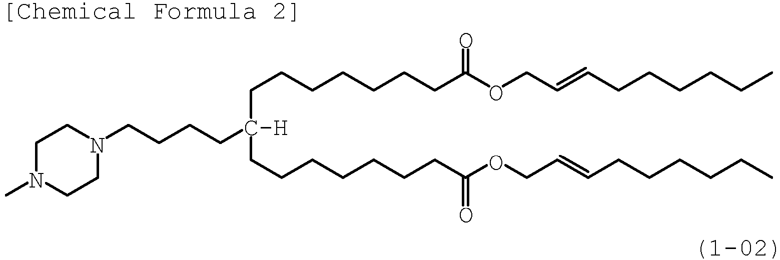

In a case where the lipid membrane 1 containing the lipid compound A described above is used, it is possible to increase an amount of nucleic acid contained and increase the transfection rate of nucleic acid. In addition, cell death rate of the transfected cells can also be reduced. In particular, use of FFT10 and FFT20 is preferable because the amount of nucleic acid contained and the transfection efficiency of nucleic acid are particularly excellent.

The lipid membrane 1 preferably further contains a lipid that suppresses aggregation by adjusting surface charge of the lipid particle 10. Such lipids are, for example, PEG-modified lipids, in particular polyethylene glycol dimyristoyl glycerol (DMG-PEG), maleimide-DMG-PEG, polyamide oligomers derived from omega-amino(oligoethylene glycol)alkanoic acid monomers (US 6,320,017), monosialogangliosides, and are preferably contained in an amount of about 1% to about 5% (molar ratio) to the total lipids in the lipid membrane 1.

As other lipids, the lipid membrane 1 may contain, for example, a lipid having relatively low toxicity for adjusting toxicity, a lipid having a functional group that binds a ligand to the lipid particle 10, and lipid sterol (for example, cholesterol) for suppressing leakage of contained substances. In particular, it is preferable that cholesterol is contained in the lipid membrane 1.

Further, the lipid membrane 1 of the lipid particle 10 preferably contains 50% or more of the cationic lipid as its composition. For example, in a case where the lipid membrane 1 includes FFT10 and FFT20, DOPE and/or DOTAP, cholesterol, DMG-PEG and/or maleimide-DMG-PEG, it is preferable that FFT10, FFT20 and DOTAP are contained in a total amount of 50% or more as a lipid composition (molar ratio).

Further, in the lipid membrane 1, it is preferable that FFT10 and FFT20 are contained in equal proportions to each other in the lipid composition (molar ratio), and a composition ratio of each is 10% to 30%. Specifically, the ratio of FFT10 and FFT20 is preferably 10%, 15%, 20%, 25%, or 30%.

Furthermore, in the lipid composition (molar ratio) of the lipid membrane, DOTAP is preferably 10% or more, and is equal to or less than the ratio of each of FFT10 and FFT20. Specifically, the ratio of DOTAP is preferably 10% in a case where the ratio of each of FFT10 and FFT20 is 10%, and the ratio of DOTAP is preferably 10%, 15%, or 20% in a case where the ratio of each of FFT10 and FFT20 is 20%.

As described above, in a case where the lipid membrane 1 of the lipid particle 10 contains the lipid compounds of FFT10 and FFT20, DOPE and/or DOTAP, cholesterol, and DMG-PEG or maleimide-DMG-PEG, the amount of nucleic acid contained and the transfection efficiency of nucleic acid are particularly excellent, which is preferable.

In particular, when the lipid membrane 1 has the lipid composition (A) or (B) shown in Table 1 below, the transfection efficiency of nucleic acid into a CD209 positive cell described later can be further enhanced. [Table 1]

**Table 1**

| Lipid composition | FFT10 | FFT20 | DOPE | DOTAP | DMG-PEG | Cholesterol |
|---|---|---|---|---|---|---|
| (A) | 20 | 20 | 0 | 10 | 48 | 2 |
| (B) | 20 | 20 | 10 | 10 | 36 | 4 |

| | | | | | | |
|---|---|---|---|---|---|---|
| (Unit: %, molar fraction) | | | | | | |

### (Nucleic acid)

The first nucleic acid 2 is, for example, a single-stranded or double-stranded cyclic, linear or branched nucleic acid. The first nucleic acid 2 is, for example, DNA, RNA, PNA, or any derivative thereof. The derivative is one obtained by inserting a nucleotide analog into DNA, RNA or PNA, one obtained by modifying any end of DNA, RNA or PNA with a label or a functional group, or the like.

In a case where the first nucleic acid 2 is RNA, it is preferably modified to be resistant to degradation. For example, the modification may be a known modification that prevents RNA from being degraded by RNase or the like. Such modifications are, for example, use/transfection of modified nucleotides/artificial nucleotides into RNA, use/addition of non-natural sequences, or addition of natural/non-natural CAP structures.

Examples of the modified nucleotide include pseudouridine, 5-methylcytidine, 1-methylpseudouridine, 5-methoxyuridine, and 1-methyl adenosine. Examples of the artificial nucleotide include a bridged nucleic acid (BNA), a locked nucleic acid (LNA), or a peptide nucleic acid (PNA).

The non-natural sequence is, for example, an artificially produced base sequence that does not exist in nature, and is, for example, a random base sequence, a hybrid sequence of a natural/non-natural amino acid and a nucleic acid, or the like. The non-natural sequence is preferably added, for example, to the end of RNA.

Examples of the natural CAP structure include CAP0 (m7GpppN) and CAP1 (m7GpppNm). Examples of the non-natural CAP structure include anti-reverse cap analog (ARCA) or LNA-guanosine. The non-natural CAP structure is preferably added, for example, to the 5' end of RNA.

The first nucleic acid 2 is a nucleic acid containing a CAR antigen gene. The CAR antigen in the present disclosure is, for example, a protein, a sugar chain, or a glycolipid that can be expressed on the surface of a CD209 positive cell described later so as to bind to a CAR expressed in an immune cell. A gene encoding a protein generally known as a CAR antigen may be used as a CAR antigen gene, or a gene encoding a protein modified to an extent that it has a similar function, a fusion protein to which an additional domain is added, or a novel protein having a similar function may be used.

Here, an example of the CAR in the present disclosure will be described in detail. The CAR includes, for example, an extracellular domain, a transmembrane domain, and an intracellular domain.

### (a) Extracellular domain

The extracellular domain is a domain that is located on an outer surface of the cell membrane of the T cell and specifically binds to a target of the CAR. For example, the extracellular domain includes an antigen-binding fragment of an anti-target monoclonal antibody, such as an scFv fragment. As the monoclonal antibody, for example, an antibody from a rodent (mouse, rat, rabbit, etc.), a human antibody or a humanized antibody, or an antigen-binding site thereof can be used. An scFv fragment is a structure in which a light chain variable region (VL) and a heavy chain variable region (VH) of an immunoglobulin are linked via a linker. As the linker, for example, a peptide linker including a peptide in which amino acids are linearly linked can be used. The peptide linker is, for example, a linker including glycine and serine (for example, a GGS linker or GS linker). For example, a linker having 5 to 25 amino acid residues can be used. Alternatively, in a case where a tumor-specific antigen that is the target of the CAR is a receptor, a ligand that binds to the receptor or the like may be used as the extracellular domain.

The type and configuration of the extracellular domain is selected depending on the type of target of the CAR. The target of the CAR in the present arrangement is the CAR antigen described above.

### (b) Transmembrane domain

The transmembrane domain is a domain located between the extracellular domain and an intracellular signaling domain in the cell membrane of the T cell. As the transmembrane domain, CD28, CD3ε, CD8α, CD3, CD4, 4-1BB, or the like can be used. Alternatively, it is also possible to use a transmembrane domain including an artificially constructed polypeptide.

### (c) Intracellular signaling domain

The intracellular signaling domain is a domain located inside the cell membrane of the T cell, and transmits signals necessary for activation of the T cell when the extracellular domain binds to the target antigen. The intracellular signaling domain includes, for example, a domain (first domain) for transmitting signals via a TCR complex. As the first domain, CD3ζ, FcεRIγ, or the like can be used. Preferably, CD3ζ is used.

Furthermore, the intracellular signaling domain may further include a domain (second domain) for transmitting costimulatory signals. As the second domain, for example, an intracellular domain of a costimulatory molecule such as CD28, 4-1BB(CD137), CD2, CD4, CD5, CD134, OX-40, or ICOS can be used. Preferably, CD28 or 4-1BB is used.

Each of the first domain and the second domain may include one of the elements listed above, or may have a configuration in which a plurality of the same or different elements are linked in a tandem manner. The order in which the first domain and the second domain are linked is not particularly limited, but it is preferable to dispose the second domain on a side of the transmembrane domain. The first domain and the second domain may be directly linked to each other, or a linker may be interposed therebetween. As the linker, for example, a peptide linker including a peptide in which 2 to 15 amino acids are linearly linked can be used.

### (d) Other elements

The CAR may include other elements. As other elements, for example, a leader sequence (signal peptide) that promotes CAR secretion, for example, a leader sequence of a GM-CSF receptor can be used. Further, a spacer domain may be disposed between the extracellular domain and the transmembrane domain. The spacer domain may facilitate binding of the CAR to the target. As the spacer domain, for example, an Fc fragment of human IgG (for example, human IgG1 and human IgG4) can be used. Alternatively, a part of the extracellular domain of CD28, a part of the extracellular domain of CD8α, or the like can be used as the spacer domain. Note that, a spacer domain can also be provided between the transmembrane domain and the intracellular signaling domain.

The CAR gene is, for example, a gene corresponding to each of the above-described domains linked in an appropriate order. A CAR gene sequence may include a full length of any of the above-described CAR genes, or may include a part thereof.

The CAR antigen in the present disclosure is typically an antigen associated with tumor cells or an antigen specific for a tumor. The antigen associated with tumor cells refers to an antigen that is observed to be significantly or notably expressed (that is, specifically or selectively expressed) in, for example, tumor cells, cells in a surrounding tissue thereof, or a living body with tumor cells as compared with cells other than a tumor. The CAR antigen may be any antigen that is a target of the CAR (that is, compatible with the type of CAR of interest) and is commonly known as a tumor-associated antigen or a tumor-specific antigen. Note that, the tumor-associated antigen or the tumor-specific antigen may be an antigen present in an extracellular matrix, or a protein containing a mutation identified by genome analysis and/or a suggestive expression study of a tumor.

In a case where the above-mentioned tumor is, for example, B cell lymphoma, multiple myeloma, retinoblastoma, pulmonary small cell tumor, central nervous system tumor, or the like, examples of the CAR antigen include CD19 antigen, CD20 antigen, GD2 antigen, CD22 antigen, CD30 antigen, CD33 antigen, CD44variant7/8 antigen, CEA antigen, Her2/neu antigen, MUC1 antigen, MUC4 antigen, MUC6 antigen, IL-13 receptor-alpha2, an immunoglobulin light chain, PSMA antigen, or VEGF receptor 2. Alternatively, in a case where the above-mentioned tumor is a leukemia stem cell, a leukemia precursor cell, a leukemia cell, or the like of a myeloid tumor, it is also possible to use GM-CSF that is a ligand of a GM-CSF (granulocyte monocyte colony stimulating factor) receptor as the CAR antigen. Alternatively, in the case of neuroblastoma, breast cancer, cervical cancer, uterine cancer, ovarian cancer, melanoma, astrocytoma, Ewing's sarcoma, glioblastoma, retinoblastoma, rhabdomyosarcoma, nonsmall cell lung cancer, prostate cancer, and urothelial cancer, it is also possible to use fragments thereof that bind to extracellular ligand-binding regions of FAM150A, FAM150B, and ALK as the CAR antigen.

In addition, depending on the type of tumor of interest, CAR antigens may include EphB4, EGFR variant 3, EphA2, EphB2, EGFR, GD2, Glypican-3,5T4,8H9, αvβ6 integrin, B cell maturation antigen (BCMA), B7-H3, B7-H6, CAIX, CA9, κ light chain, CD38, CD44, CD44 variant 6, CD70, CD116, CD123, CD138, CD171, CEA, CSPG4, EGP2, EGP40, EPCAM, ERBB3, ERBB4, ErbB3/4, FAP, FAR, FBP, fetal AchR, folate receptor α, GD3, HLA-AI MAGE A1, HLA-A2, IL11Ra, IL13Ra2, KDR, Lambda, Lewis Y, MCSP, mesoserine, NCAM, NKG2D ligand, NY-ESO-1, PRAME, PSCA, PSC1, ROR1, Sp17, SURVIVIN, TAG72, TEM1, TEM8, VEGR receptor 2, carcinoembryonic antigen, HMW-MAA, VEGF receptor, fibronectin, tenascin, ALK (anaplastic lymphoma kinase), or an antigen present in an extracellular matrix such as CEA in a tumor necrotic region. Furthermore, it may be the full length or a part of a base sequence obtained by combining a plurality of these genes.

### (Nucleic acid condensing peptide)

A nucleic acid condensing peptide 3 is a peptide capable of further reducing the volume occupied by nucleic acids by condensing more nucleic acids, and can efficiently contain a large amount of nucleic acid within the lipid particle 10. As such a peptide, for example, a cationic peptide is preferably used. The cationic peptide can condense nucleic acids, for example, by entering into spiral gaps of anionic nucleic acids to shorten the gaps.

A preferred nucleic acid condensing peptide 3 is, for example, a peptide containing cationic amino acids in an amount of 45% or more of the total. A more preferred nucleic acid condensing peptide 3 has RRRRRR (a first amino acid sequence) at one end and has the sequence RQRQR (a second amino acid sequence) at the other end. Then, between both amino acid sequences, 0 or 1 or more intermediate sequences including RRRRRR or RQRQR are contained. Further, two or more neutral amino acids are contained between two adjacent sequences among the first amino acid sequence, the second amino acid sequence, and the intermediate sequence. The neutral amino acid is, for example, G or Y.

The nucleic acid condensing peptide 3 preferably has the following amino acid sequence.
RQRQRYYRQRQRGGRRRRRR (SEQ ID NO:1)
RQRQRGGRRRRRR (SEQ ID NO:2)

Such a nucleic acid condensing peptide 3 can efficiently condense nucleic acids by cationic arginine, and weaken anionic properties of the nucleic acid, making it possible to efficiently contain nucleic acids in the lipid particle 10. Furthermore, since the nucleic acid condensing peptide 3 efficiently dissociates nucleic acids within the cell, the first nucleic acid transfected into the cell can be efficiently expressed in the CD209 positive cell.

Alternatively, the nucleic acid condensing peptide 3 has RRRRRR (a third amino acid sequence) at one end and RRRRRR (a fourth amino acid sequence) at the other end. Then, between both amino acid sequences, 0 or 1 or more intermediate sequences including RRRRRR or RQRQR are contained. Further, two or more neutral amino acids are contained between two adjacent sequences among the third amino acid sequence, the fourth amino acid sequence, and the intermediate sequence.

Such a nucleic acid condensing peptide 3 preferably has the following amino acid sequence.
RRRRRRYYRQRQRGGRRRRRR (SEQ ID NO:3)

Such a nucleic acid condensing peptide 3 has strong cationic properties at both ends and high binding affinity to nucleic acids. Therefore, it is possible to more efficiently condense nucleic acids and contain more nucleic acids within the lipid particle 10. As a result, the amount of nucleic acid remaining outside the lipid particle 10 is reduced, thereby preventing aggregation of the lipid particles 10 with each other, and thus the lipid particle 10 is more likely to be taken up into a cell.

Furthermore, the nucleic acid condensing peptide 3 having the following amino acid sequence can also be used in combination with any of the nucleic acid condensing peptides 3 described above.
GNQSSNFGPMKGGNFGGRSSGPYGGGGQYFAKPRNQGGY(M9) (SEQ ID NO:4)

This peptide can further condense nucleic acid aggregates condensed with the nucleic acid condensing peptide 3 described above. Therefore, it is possible to obtain lipid particles with a smaller particle size. The smaller the particle size, the easier it is for the lipid particle to be taken up into the CD209 positive cell, making it possible to more efficiently transfect nucleic acids into the CD209 positive cell. For example, the first nucleic acid 2 can be condensed by stirring and mixing the first nucleic acid 2 with the nucleic acid condensing peptide 3 before being contained in the lipid particle 10.

It is more preferable to use the nucleic acid condensing peptide 3 from a viewpoint of achieving the above-described effects, but the nucleic acid condensing peptide 3 may not be used depending on the type of nucleic acid to be used, culture conditions of the cell, or the like.

The lipid particle 10 may contain further components in addition to the first nucleic acid 2 and the nucleic acid condensing peptide **3.** For example, a compound that modulates expression of nucleic acids in a cell, such as retinoic acid, cyclic adenosine monophosphate (cAMP), or ascorbic acid, may be contained. Alternatively, it is also possible to contain, for example, peptides, polypeptides, cytokines, growth factors, apoptosis factors, differentiation-inducing factors, other cell surface receptors and ligands thereof, and the like.

The lipid particle 10 can be produced, for example, using a known method used when a small molecule is enclosed in a lipid particle or the like. Examples of the known method include the Bangham method, an organic solvent extraction method, an ethanol injection method, a surfactant removal method, and a freeze-thaw method. In a case where the organic solvent extraction method is selected, for example, an aqueous buffer containing a component to be contained, such as the first nucleic acid 2, is added to a mixture obtained by containing the material of the lipid membrane 1 in an organic solvent such as alcohol, and the mixture is stirred and suspended, whereby the lipid particle 10 can be produced. In a case where the ethanol injection method is selected, for example, ethanol obtained by containing the material of the lipid membrane 1 is added to an aqueous buffer containing a component to be contained, such as the first nucleic acid 2, and the mixture is stirred and suspended, whereby the lipid particle 10 can be produced. The ratio of the amounts of the nucleic acids contained in the lipid particle 10 can be easily adjusted by changing the ratio of the amounts of the nucleic acids in the aqueous buffer. The amount of nucleic acid contained can be confirmed using, for example, commercially available DNA and RNA quantification kits.

The average particle size of the lipid particle 10 is, for example, about 50 nm to about 300 nm, preferably about 50 nm to about 200 nm. For example, the particle size can be reduced by ultrasonic treatment. Further, it is also possible to adjust the size of the lipid particle 10 by allowing the particle to pass through a polycarbonate membrane or a ceramic membrane. Note that, the average particle size of the lipid particle 10 can be measured by, for example, a Zetasizer using a dynamic light scattering method.

The lipid particle of the arrangement described above may be provided as a composition contained in an appropriate carrier. The carrier is, for example, water, a saline solution such as physiological saline, an aqueous glycine solution, or a buffer solution such as HEPES.

The composition containing lipid particles may further contain a substance that improves storage stability. The substance that improves storage stability is not limited, and is, for example, a glycoprotein such as albumin, lipoprotein, apolipoprotein, and globulin. Specifically, the substance that improves storage stability is a pH adjusting agent, a buffering agent, a tonicity adjusting agent, or the like. Alternatively, it may be a participating agent that is pharmaceutically acceptable and brings a pharmaceutical composition closer to a physiological state, such as sodium acetate, sodium lactate, sodium chloride, potassium chloride, or calcium chloride. Alternatively, it may be a lipophilic free radical quencher such as α-tocopherol that suppresses damage caused by free radicals. Furthermore, it is a lipid protective agent such as water-soluble chelators like ferrioxamine for suppressing lipid peroxidation damage and improving storage stability. The substance that improves storage stability is preferably added to a solution or the like containing the lipid particle 10 after the lipid particle 10 is formed.

A composition containing lipid particles may be sterilized by common methods. Further, the composition may also be provided as a liquid or as a dried powder. The powder composition can be used, for example, by being dissolved in a suitable liquid.

The concentration of the lipid particle 10 contained in the composition is not limited, but is preferably 0.01 to 30% by mass, and preferably 0.05 to 10% by mass. The concentration is appropriately selected according to the purpose.

### · Method for producing CAR antigen-expressing cells

The method for producing a CAR antigen-expressing cell of the first arrangement includes a preparation step (S1) of preparing a CD209 positive cell and the lipid particle described above, and a gene transfer step (S2) of bringing the prepared CD209 positive cell into contact with the lipid particle.

Here, the CD209 positive cell in the present disclosure is a cell in which CD209 is present on the cell surface (more specifically, a cell expressing CD209, a C-type lectin molecule, as a type II transmembrane pattern recognition receptor), and examples thereof include a dendritic cell and a macrophage, and the CD209 positive cell in the present disclosure is preferably a dendritic cell. The dendritic cell includes an immature dendritic cell as found in peripheral tissues, a mature dendritic cell as found in lymphatic tissues, and the like, but the CD209 positive cell of the present disclosure is more preferably an immature dendritic cell.

The CD209 positive cell in the present arrangement may be a cell collected from a subject to which the CAR-T cells finally obtained by a proliferation method of a second arrangement are administered, that is, a cell population derived from an autologous cell population. Alternatively, the CD209 positive cell may be a cell collected from a cell population derived from other family or a commercially available cell population, or may be a cell differentiated from hematopoietic stem cells or a cell differentiated from iPS cells.

The gene transfer step (S2) can be performed, for example, by mixing a culture solution containing the CD209 positive cells prepared in the preparation step (S1) with a solution containing lipid particles. In other words, the production method of the first arrangement can be performed by a simple operation of bringing the lipid particle of the first arrangement in which the lipid membrane exhibits the lipid composition as described above into contact with the CD209 positive cell. By the production method of the first arrangement, the gene can be transferred into the CD209 positive cell with high efficiency, and thus the CAR antigen-expressing cell can be produced with high efficiency.

In the method for producing a CAR antigen-expressing cell of the first arrangement, the preparation step (S1) and the gene transfer step (S2) may be performed continuously, but other steps for producing a CAR antigen-expressing cell may be included in between. For example, between the preparation step (S1) and the gene transfer step (S2), a pre-culture step for enhancing activity by culturing the CD209 positive cell in advance may be included. Further, after the gene transfer step (S2), a post-culture step of culturing the CD209 positive cell in contact with the lipid particle may be included. Note that, the pre-culture step of the CD209 positive cell as a post-step of the preparation step (S1) and the post-culture step of the CD209 positive cell as a post-step of the gene transfer step (S2) have been described, but the preparation step (S1) may be interpreted as including the pre-culture step, and the gene transfer step (S2) may be interpreted as including the post-culture step.

### [Second Arrangement]

### · Methods for proliferating CAR-T cells

As a further application, the lipid particle described in the first arrangement can also be used in a method for proliferating CAR-T cells including production of a CAR antigen-presenting cell. Hereinafter, an example of a method for proliferating CAR-T cells using the lipid particle of the first arrangement will be described with reference to the drawings.

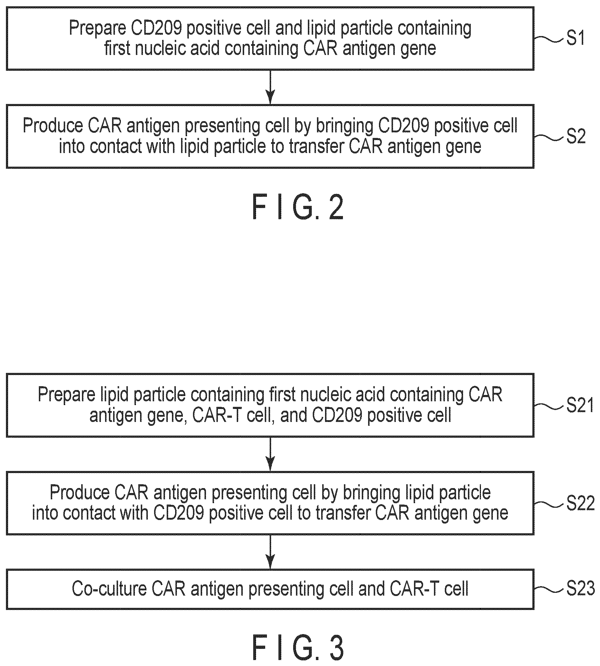

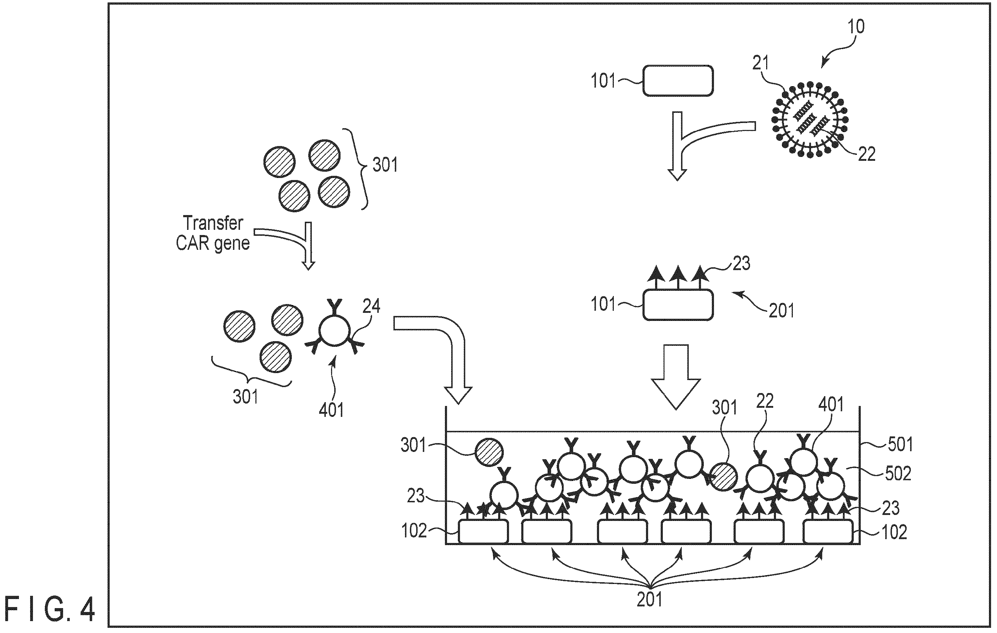

As shown in FIG. 3, the method for proliferating CAR-T cells of the second arrangement includes a preparation step (S21) of preparing the lipid particle of the first arrangement, a CAR-T cell, and a CD209 positive cell, a preparation step (S22) of producing a CAR antigen-presenting cell by bringing the lipid particle of the first arrangement into contact with the CD209 positive cell to transfer a CAR antigen gene, and a co-culture step (S23) of co-culturing the CAR-T cell and the CAR antigen-presenting cell. Hereinafter, each step will be described in detail with reference to FIGS. 3 and 4.

The CAR-T cell prepared in the preparation step (S21) may be produced in the preparation step (S21). For example, the preparation step (S21) may include a step of preparing a cell population 301 containing T cells and producing a CAR-T cell 401 from the cell population 301.

T cells include CD4-positive CD8-negative T cells, CD4-negative CD8-positive T cells, T cells prepared from iPS cells, αβ-T cells, γδ-T cells, precursor cells thereof, and the like. A variety of cell populations can be used as long as they contain such T cells. For example, the cell population 301 containing T cells may be peripheral blood mononuclear cells (PBMCs) collected from peripheral blood. The cell population 301 containing T cells is more preferably a cell population collected from a subject to which the CAR-T cells finally obtained by the proliferation method of the second arrangement are administered (that is, an autologous cell population). However, a cell population derived from other family, a commercially available cell population, and the like can also be used. Further, a cell population subjected to a treatment of separating unnecessary cells from the collected or obtained cell population may be used.

Note that, it is preferable to culture the cell population 301 containing T cells in an appropriate culture medium and culture conditions before the production of a CAR-T cell. By culturing the cells, cell activity is enhanced, and subsequent transfer of a CAR gene can be performed with high efficiency.

Subsequently, as shown in FIG. 4, the CAR gene is transferred into a T cell, and the CAR is expressed to produce a CAR-T cell 401. The transfer of the CAR gene into the T cell may be performed by any tool or method generally known as a gene transfer tool or a gene transfer method into a cell. Further, as long as the CAR is expressed, the CAR gene may be integrated into a genome of the T cell or not, but it is more preferable to integrate the CAR gene into the genome. As a gene transfer tool or a gene transfer method into a cell, for example, polycation, calcium phosphate, lipofection, a liposome, electroporation, microinjection, a particle gun, a retro/lentiviral vector, an adenoviral vector, or the like may be used. Further, a foreign gene integration tool, such as a tool using a transposon vector, a homologous recombination method, and a recombinase system, or a genome editing tool such as ZFN or CRISPR/Cas9, may be used in combination.

Hereinafter, as a CAR gene transfer tool into a T cell, a procedure for preparing a CAR-T cell in a case of using a nucleic acid transfection reagent 20 containing the nucleic acid of the CAR gene will be described. The nucleic acid transfection reagent 20 may be, for example, a lipid particle containing the nucleic acid of the CAR gene or a viral vector containing the nucleic acid of the CAR gene.

First, the nucleic acid transfection reagent 20 containing the nucleic acid of the CAR gene is brought into contact with the cell population 301 containing T cells. By this contact, the CAR gene is transferred into the T cell. This contact can be performed by, for example, a method of adding a nucleic acid transfection reagent to a suspension of the cell population 301, a method of adding beads coated with the nucleic acid transfection reagent 20 to a suspension of the cell population 301, a method of adding a suspension of the cell population 301 to a container or the like coated with the nucleic acid transfection reagent 20 on the surface, or the like. Next, the cell population 301 in contact with the nucleic acid transfection reagent 20 is cultured, whereby the CAR-T cell 401 can be produced.

The nucleic acid contained in the nucleic acid transfection reagent 20 may contain additional nucleic acids in addition to the nucleic acid containing the CAR gene. Such nucleic acids are, for example, DNA or RNA having a function to catalyze the integration of foreign DNA into the genome or having a function to modify DNA, such as methylation, demethylation, repair, and/or binding of DNA. For example, these nucleic acids may be DNA or RNA encoding a protein having the activity of the above-described modification. By containing these nucleic acids, for example, it is also possible to add the above-described modification to a CAR gene sequence transferred into the genome or its surrounding sequences, and to add further functional modifications to the T cell.

Note that, it has been described that the proliferation method of the present arrangement may include a step of producing a CAR-T cell from the cell population 301 as a separate step prior to the preparation step (S21), but the preparation step (S21) may also be interpreted as including the step of producing a CAR-T cell from the cell population 301.

Next, the preparation step (S22) will be described. In the preparation step (S22), as shown in FIGS. 3 and 4, a lipid particle 10 is brought into contact with a CD209 positive cell 101. The contact can be performed, for example, by adding a solution or composition containing the lipid particles 10 to a cell suspension in which the CD209 positive cells 101 are suspended. Alternatively, the contact may be performed by adding a solution or composition containing the lipid particles 10 to a container or the like in which the CD209 positive cells 101 are attached or fixed to the surface. Alternatively, the contact may be performed by adding a cell suspension in which the CD209 positive cells 101 are suspended to a container or the like in which the lipid particles 10 are attached or fixed to the surface.

The lipid particle 10 is taken up into the CD209 positive cell 101 by endocytosis caused by contact of the lipid particle 10 or membrane fusion between the lipid particle and the cell membrane of the CD209 positive cell 101. A first nucleic acid 22 is released into the CD209 positive cell 101 from the lipid particle 10 taken up into the CD209 positive cell 101, whereby the first nucleic acid 22 can be transfected into the CD209 positive cell 101. When the transfected first nucleic acid 22 expresses a CAR antigen 23 in the CD209 positive cell 101, a CAR antigen-expressing cell 201 can be produced.

Next, in a co-culture step (S23), the CAR-T cell 401 prepared in the preparation step (S21) and the CAR antigen-expressing cell 201 prepared in the preparation step (S22) are co-cultured. For example, the co-culture can be performed by adding the CAR-T cell 401 and the CAR antigen-expressing cell 201 to a container 501 containing a basic medium 502. As the basic medium 502 used for co-culture, a medium suitable for culturing both the CAR-T cell 401 and the CAR antigen-presenting cell 201 may be used. The culture conditions are preferably conditions suitable for survival and proliferation of both the CAR-T cell 401 and the CAR antigen-presenting cell 201.

In the co-culture step (S23), the CAR antigen-expressing cell 201 may be directly or indirectly attached or fixed to one surface of a solid phase. The solid phase is, for example, a container 501 made of metal, resin, gel, fiber, or the like, but is not necessarily required to have a container shape, and may be a plate, a sheet, or the like. In a case where such a solid phase is used in the method for proliferating CAR-T cells, the co-culture step (S23) can be simply performed by adding a cell suspension containing the CAR-T cells 401 onto the surface of the solid phase to which the CAR antigen-expressing cells 201 are attached or fixed. Further, in a subsequent recovery step, it is possible to easily recover the proliferated CAR-T cells 401 while leaving the CAR antigen-expressing cells 201 fixed on the solid phase by replacing the basic medium 502.

In the co-culture step (S23), the CAR antigen-expressing cell 201 presents an antigen, thereby activating the CAR-T cell 401. Therefore, in the method of the present arrangement, the CAR antigen-expressing cell is also referred to as a "CAR antigen-presenting cell". As shown in examples described later, the use of a cell derived from CD209 positive cell as the CAR antigen-expressing cell has higher proliferation efficiency of CAR-T cells than the use of the CAR antigen-presenting cell derived from other cells or cell groups (for example, PBMCs).

The CAR antigen-presenting cell 201 in the present arrangement is a CD209 positive cell expressing a CAR antigen, and such a CD209 positive cell generally plays an important role in innate immunity detection of a pathogen and subsequent activation of adaptive immunity in a living body. More specifically, CD209 positive cells in a living body (for example, dendritic cells, macrophages, and the like) take in and degrade pathogens by phagocytosis, and present a target antigen to T cells, thereby initiating an adaptive immune response to induce the activation and differentiation of T cells, as well as the differentiation of regulatory T cells. Furthermore, CD209 positive cells in a living body also have a function of secreting cytokines, interferons, or growth factors that enhance and regulate an immune response. Therefore, by subjecting the CD209 positive cell to co-culture as a CAR antigen-presenting cell, CAR-T cells can be proliferated more efficiently than when other cells or cell groups (for example, PBMCs) are used. The reason for this is considered to be that the CD209 positive cell, being an antigen presenting cell inherent in a living body, is more likely to stimulate the CAR-T cell.

Further, in order to transform other cells or cell groups other than the CD209 positive cell into the CAR antigen-presenting cell, it is also necessary to transfer other genes (for example, costimulatory factors). On the other hand, when only a target CAR antigen gene is transferred, the CD209 positive cell functions as a CAR antigen-presenting cell in the proliferation method of the present arrangement. Therefore, the proliferation method of the present arrangement is preferable in that the gene transfer step can be performed with fewer elements than the conventional method, thereby reducing costs and labor, and preventing deterioration in quality and production amount due to contamination.

Furthermore, in the proliferation method of the present arrangement, by using the lipid particle with a specific lipid composition as described in the first arrangement, a CAR antigen-presenting cell group with a significantly high expression rate of the CAR antigen gene can be produced. By using this CAR antigen-presenting cell group, it is possible to further activate CAR-T cells and increase a proliferation rate of the CAR-T cells. In other words, by applying the method of the present arrangement that includes a co-culture step with the CAR antigen-presenting cell to a cell population containing CAR-T cells, the content of CAR-T cells in the cell population can be improved.

The proliferation method of the present arrangement may further include a step of recovering the obtained CAR-T cells after the co-culture step (S23). For example, the CAR-T cells 401 in the basic medium 502 can be recovered by general treatment such as pipetting or centrifugation.

As a further arrangement, the CAR-T cells proliferated by the method of the present arrangement may be used to produce a cell preparation. The cell preparation may contain CAR-T cells in a therapeutically effective amount. The cell preparation may contain an agent for protecting CAR-T cells, such as dimethyl sulfoxide (DMSO) or serum albumin, an agent for preventing the proliferation of microorganisms such as antibiotics, an agent for activating, proliferating, or inducing differentiation of cells, such as vitamins, cytokines, growth factors, or steroids, and the like.

An administration route of the CAR-T cells or the cell preparation obtained by the proliferation method of the present arrangement is not particularly limited. For example, the cells are administered by intravenous injection, intraarterial injection, intraportal injection, intradermal injection, subcutaneous injection, intramuscular injection, or intraperitoneal injection. Alternatively, the administration method may be topical administration. In that case, for example, the cells are directly injected into a target tissue or organ. An administration schedule may be selected in consideration of the sex, age, weight, disease state, or the like of the subject (patient), and may be single administration, or may be continuous or periodic multiple administrations.

### [Third Arrangement]

### · Kit

The kit of the third arrangement is a kit used in the method for proliferating CAR-T cells, and includes lipid particles for producing a CAR antigen-presenting cell described in the first arrangement. Furthermore, the kit of the third arrangement may include a nucleic acid transfection reagent for transferring a CAR gene into CAR-T cells described in the second arrangement. However, in the kit of the third arrangement, the lipid particles of the first arrangement and the nucleic acid transfection reagent of the second arrangement are not provided in a mixed state (that is, not provided as a composition), but are preferably included in the kit as a separate reagent.

The kit may further contain other components in the above composition or as a separate composition. Other components include a cell culture medium and/or antibodies, peptides, and the like for activating T cells. A substance that improves the storage stability of lipid particles and/or a nucleic acid transfection reagent may be contained, and for example, any of the substances that improve the storage stability exemplified in the first arrangement may be similarly contained.

The kit may include a solid phase having a surface to which CD209 positive cells can be directly or indirectly attached or fixed. The solid phase is, for example, a container made of metal, resin, gel, fiber, or the like, but is not necessarily required to have a container shape, and may be a plate, a sheet, or the like. In the kit, lipid particles (or a nucleic acid transfection reagent) may be provided by being directly or indirectly attached or fixed to one surface of the solid phase.

Direct attachment or fixation can be performed, for example, by directly applying lipid particles or a nucleic acid transfection reagent to the surface of the solid phase and drying the lipid particles or the nucleic acid introduction reagent. Indirect attachment or fixation can be performed, for example, by applying lipid particles or a nucleic acid transfection reagent onto the solid phase via a peptide, a resin, an antibody or the like, and drying the lipid particles or the nucleic acid transfection reagent.

In a case where a kit including such a solid phase is used in the method for proliferating CAR-T cells, a gene transfer step can be easily performed by adding a cell suspension containing various types of cells onto the surface of the solid phase to which lipid particles (or a nucleic acid transfection reagent) are attached or fixed. For example, in a case where lipid particles are releasably attached or fixed to the surface of the solid phase, the lipid particles can be released into the cell suspension by adding a cell suspension containing CD209 positive cells to the surface of the solid phase, and can be brought into contact with the CD209 positive cells. Similarly, in a case where a nucleic acid transfection reagent is releasably attached or fixed to the surface of the solid phase, the nucleic acid transfection reagent can be released into the cell suspension by adding a cell suspension containing CAR-T cells to the surface of the solid phase, and can be brought into contact with the CAR-T cells.

### [Examples]

Hereinafter, examples of producing and using lipid particles, a CAR antigen-presenting cell, and eventually a CAR-T cell by the method of the arrangements will be described. However, the arrangements of the present invention are not limited to the following examples.

### Example 1. Selection of lipid composition of lipid particles suitable for gene transfer into dendritic cells

In order to determine the lipid composition of lipid particles suitable for gene transfer into dendritic cells, an experiment of Example 1 was performed according to the following procedure.

### · Preparation of lipid particles

As a nucleic acid to be contained in the lipid particle, mRNA encoding GFP (green fluorescent protein) was used. A solution containing this mRNA and an ethanol-dissolved lipid solution having lipid compositions (1) to (19) were mixed, then further mixed with 10mM HEPES (pH7.3), and washed and concentrated by centrifugal ultrafiltration, so that a total of 19 kinds of solutions containing lipid particles exhibiting different lipid compositions were obtained. [Table 2]

**Table 2**

| Lipid composition | FFT10 | FFT20 | DOPE | DOTAP | DMG-PEG | Cholesterol |
|---|---|---|---|---|---|---|
| (1) | 40 | 0 | 10 | 20 | 26 | 4 |
| (2) | 20 | 0 | 10 | 20 | 46 | 4 |
| (3) | 20 | 0 | 0 | 20 | 54 | 6 |
| (4) | 20 | 0 | 20 | 20 | 36 | 4 |
| (5) | 20 | 0 | 20 | 0 | 54 | 6 |
| (6) | 0 | 20 | 10 | 10 | 56 | 4 |
| (7) | 0 | 20 | 20 | 10 | 44 | 6 |
| (8) | 0 | 20 | 20 | 20 | 34 | 6 |
| (9) | 0 | 40 | 0 | 20 | 36 | 4 |
| (10) | 0 | 40 | 20 | 10 | 28 | 2 |
| (11) | 30 | 10 | 0 | 20 | 34 | 6 |
| (12) | 30 | 10 | 10 | 10 | 38 | 2 |
| (13) | 30 | 10 | 20 | 0 | 34 | 6 |
| (14) | 20 | 20 | 0 | 10 | 48 | 2 |
| (15) | 20 | 20 | 10 | 10 | 36 | 4 |
| (16) | 20 | 20 | 20 | 0 | 34 | 6 |
| (17) | 10 | 30 | 0 | 10 | 46 | 4 |
| (18) | 10 | 30 | 10 | 0 | 44 | 6 |
| (19) | 10 | 30 | 10 | 10 | 36 | 4 |

| | | | | | | |
|---|---|---|---|---|---|---|
| (Unit: %, molar fraction) | | | | | | |

### Preparation of dendritic cells

DCs (dendritic cells) were separated from PBMCs (peripheral blood mononuclear cells) by an adhesion method and cultured. Frozen PBMCs (manufactured by Lonza) were thawed, 200 µL of PBMCs (7.0×10⁶ cells/mL) suspended in ALyS705 (Cell Science & Technology Institute, Inc.) was then added to a 96 well culture plate, and the plate was placed in an incubator at 37°C in a 5%CO₂ atmosphere for four hours. The culture plate was taken out from the incubator, the medium was removed from the well, and then the plate was washed three times with phosphate buffered saline (PBS) to remove non-adherent cells. After washing, 200 µL of ALyS705 (containing IL4:10 ng/mL, GM-CSF: 10 ng/mL) was added, and cell culture was continued at 37°C in a 5%CO₂ atmosphere.

### · Transfection of nucleic acid into dendritic cells by lipid particles

For each of a total of 19 kinds of solutions containing lipid particles exhibiting different lipid compositions, GFP mRNA was added to each well of the culture plate containing dendritic cells. In other words, a solution containing lipid particles was dispensed such that each well contained lipid particles exhibiting a composition of any one of the lipid compositions (1) to (19), that is, different wells contained lipid particles with different lipid compositions. After dispensing, the culture plate was placed in an incubator at 37°C in a 5%CO₂ atmosphere to culture the dendritic cells.

### · Measurement of gene transfer activity in dendritic cells

After 24 hours from the culture, the culture plate was taken out from the incubator, and the GFP fluorescence intensity and cell proliferation activity of the cells were measured. The GFP fluorescence intensity was measured with a fluorescence microplate reader (Infinite F200 PRO, Tecan). After measurement of the fluorescence intensity, the cell proliferation activity was measured with Premix WST-1 Cell Proliferation Assay System (manufactured by Takara Bio Inc.). ALyS705 added with a Premix WST-1 solution was added to each well of a culture plate, and then the plate was placed in an incubator at 37°C in a 5%CO₂ atmosphere, and 30 minutes later, the culture plate was taken out from the incubator, and the absorbance of the well was measured. The absorbance at 450 nm and 690 nm was measured, and the value of the cell proliferation activity was a value obtained by dividing the value of the OD450nm by the value of the OD690nm. The lipid particles were evaluated using the product of the value of the fluorescence intensity and the value of cell activity as an index.

### · Results

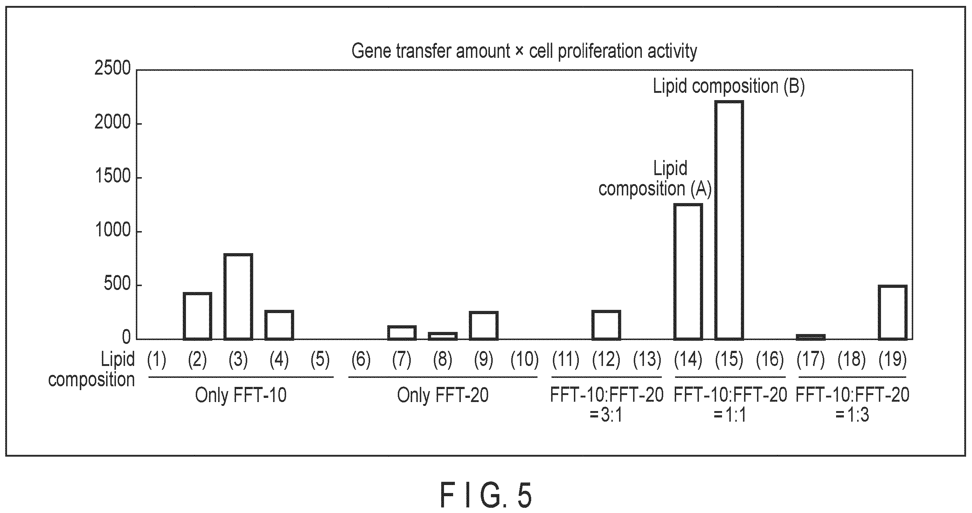

The experimental results of Example 1 are shown in FIG. 5. In FIG. 5, the value on the vertical axis represents the value obtained by integrating the GFP fluorescence intensity and the cell proliferation activity, and the value on the horizontal axis represents the composition corresponding to each of the lipid compositions (1) to (19) described in Table 2.

As shown in FIG. 5, it can be seen that when a gene is transferred using lipid particles with a lipid composition (14) or (15) in Table 2, the transfer activity of a CAR antigen gene (abundance of CAR antigen-presenting cells) is significantly higher than that when a gene is transferred using lipid particles with other lipid compositions. In other words, when the lipid particles with the lipid composition (14) or (15) in Table 2 are used, it can be said that production efficiency of CAR antigen-presenting cells is particularly excellent. Note that, the lipid composition (14) is the same as lipid composition (A) described in Table 1, and the lipid composition (15) is the same as lipid composition (B) described in Table 1.

Note that, since the numerical value obtained by integrating the GFP fluorescence intensity and the cell proliferation activity is a numerical value obtained by adding both gene transfer efficiency for lipid particles with different lipid compositions and the subsequent proliferation activity of CAR antigen-presenting cells, it can be seen that the lipid particles with the lipid composition (14) or (15) in Table 2 exhibits high performance (high gene transfer efficiency and low cytotoxicity) in gene transfer to dendritic cells.

From the above results, it can be seen that when FFT10 and FFT20 are contained in equal proportions to each other in the lipid composition (molar ratio), and the composition ratio thereof is 10% to 30%, and DOTAP is 10% or more in the lipid composition (molar ratio), and is equal to or less than the composition ratio of each of FFT10 and FFT20, the gene transfer efficiency by lipid particles is high and the cytotoxicity is low, so that the dendritic cells expressing a CAR antigen can be produced with high efficiency, which is preferable.

### Example 2. Comparison of proliferation efficiency of CAR-T Cells between liposome-DC method and AP method

In order to compare the proliferation efficiency of CAR-T cells by the proliferation method of the second arrangement (hereinafter, referred to as the "liposome-DC method") with the proliferation efficiency of CAR-T cells by the conventional proliferation method (hereinafter, referred to as the "AP method"), the experiment of Example 2 was performed according to the following procedure.

### · Preparation of dendritic cells

DCs were separated from PBMCs by an adhesion method and cultured. Frozen PBMCs (manufactured by Lonza) were thawed, and then PBMCs (3.0×10⁶ cells/well) suspended in ALyS705 (Cell Science & Technology Institute, Inc.) were added to a 24 well culture plate, and the plate was placed in an incubator at 37°C in a 5%CO₂ atmosphere for four hours. The culture plate was taken out from the incubator, the medium was removed from the well, and then the plate was washed three times with PBS to remove non-adherent cells. After washing, ALyS705(containing IL4:10 ng/mL, GM-CSF: 10 ng/mL) was added, and cell culture was continued at 37°C in a 5%CO₂ atmosphere.

### · Preparation of PBMCs

PBMCs were placed in an incubator at 37°C in a 5%CO₂ atmosphere with ALyS705 (containing IL7:10 ng/mL, IL15:5 ng/mL) added with 5% artificial serum (Cell Science & Technology Institute, Inc.), and to culture the PBMCs.

### · Production of CAR-T cells

An operation of transferring a CAR gene into the prepared PBMCs was performed to prepare a cell population containing CAR-T cells for co-culture with CAR antigen-presenting cells. In other words, the produced CAR-T cells were subjected to co-culture with CAR antigen-presenting cells in a form included in a cell population including a cell group derived from PBMCs of non-CAR-T cells. CAR-T expressing a chimeric antibody receptor that recognizes EphB4 (EphB4CAR-T) was produced. CAR-T was produced by the piggyBAC method using a DNA transferase derived from a moth. In other words, a plasmid DNA for CAR expression and a plasmid DNA for piggyBAC expression were transfected into PBMCs by an electroporation method to prepare the cells.

### · Preparation of lipid particles

Lipid particles were prepared as follows. As a nucleic acid contained in the lipid particle, mRNA encoding an EphB4 antigen (hereinafter, referred to as "EphB4-mRNA") was used. A solution containing this mRNA and an ethanol-dissolved lipid solution (that is, FFT10/FFT20/DOPE/DOTAP/cholesterol/PEG-DMG=20/20/10/10/36/4 (mol%)) were mixed, then further mixed with 10mM HEPES (pH7.3), and washed and concentrated by centrifugal ultrafiltration to obtain a solution containing lipid particles containing EphB4-mRNA.

### · Production of CAR antigen-presenting cells for use in liposome-DC method

In the liposome-DC method, the lipid particles prepared as described above were brought into contact with the dendritic cells prepared as described above to transfect EphB4-mRNA as a CAR antigen gene, thereby producing CAR antigen-presenting cells for co-culture with CAR-T cells. Specifically, lipid particles per dendritic cell were added so as to be 2.0 µg RNA/well, and the mixed liquid was placed in an incubator at 37°C in a 5%CO₂ atmosphere and cultured.

### · Production of CAR antigen-presenting cells for use in AP method

In the AP method, EphB4-plasmid DNA as a CAR antigen gene was transfected into the PBMCs prepared as described above by the electroporation method to produce CAR antigen-presenting cells for co-culture with CAR-T cells, and the CAR antigen-presenting cells were placed in an incubator at 37°C in a 5%CO₂ atmosphere and cultured.

### · Co-culture by liposome-DC method

In the liposome-DC method, the CAR antigen-presenting cells derived from dendritic cells prepared as described above and a cell population containing CAR-T cells were co-cultured. Specifically, a cell population containing CAR-T cells was added to the CAR antigen-presenting cells derived from dendritic cells one day after the transfection of EphB4-mRNA and the start of culture, and the cells were placed in an incubator at 37°C in a 5%CO₂ atmosphere to start culture.

### · Co-culture by AP method

In the AP method, the CAR antigen-presenting cells derived from PBMCs prepared as described above and a cell population containing CAR-T cells were co-cultured. Specifically, the CAR antigen-presenting cells derived from PBMCs were recovered from the cell suspension three days after the transfection of EphB4-plasmid DNA and the start of culture, and the cells were placed in an incubator at 37°C in a 5%CO₂ atmosphere together with the cell population containing CAR-T cells to start culture.

### · Measurement of abundance of T cells and CAR-T cells

The co-culture by the liposome-DC method and the co-culture by the AP method were both completed at 14 days after the start of the culture, and fluorescent immunostaining of CD3 and EphB4 CAR as T cell surface antigens was performed, and the abundance of these expressing cells was measured by FACS.

### · Results

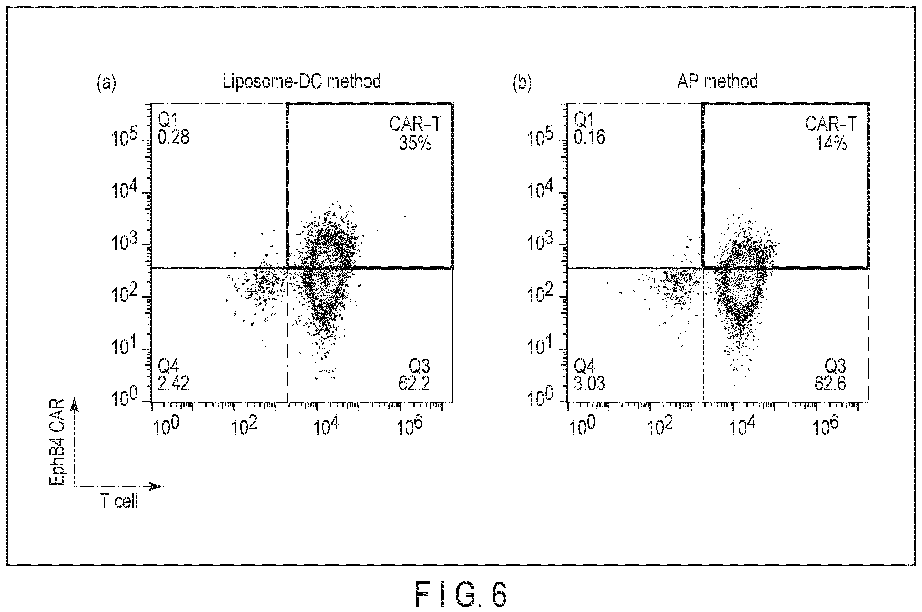

The experimental results of Example 2 are shown in FIG. 6. Part (a) of FIG. 6 shows the result of CAR-T cell proliferation by the liposome-DC method, and Part (b) of FIG. 6 shows the result of CAR-T cell proliferation by the AP method. FIG. 6 is a dot plot in which the vertical axis represents an expression level of EphB4 CAR (that is, the abundance of CAR-T cells in the cell population) and the horizontal axis represents the expression level of CD3 as a T cell surface antigen (that is, the abundance of T cells in the cell population).

From the dot plots in FIG. 6, it can be seen that CD3-positive cells (T lymphocyte cells) and EphB4 CAR-positive cells (CAR-expressing cells), that is, CAR-T cells are present in the Q2 fraction. The content of CAR-T cells in the cell population was about 14% in the case of the AP method, whereas the content of CAR-T cells in the cell population was about 35% in the case of the liposome-DC method.

From this result, it became clear that the liposome-DC method is superior to the AP method as a method for proliferating CAR-T cells, that is, the proliferation method using CAR antigen-presenting cells derived from dendritic cells is superior to the proliferation method using CAR antigen-presenting cells derived from PBMCs.

### Example 3. Effect of maleimide liposomes on gene transfer into dendritic cells

In order to verify the effect of maleimide liposome on gene transfer into dendritic cells, the experiment of Example 3 was performed according to the following procedure.

### · Preparation of lipid particles

Lipid particles were prepared as follows. As a nucleic acid contained in the lipid particle, mRNA of a CAR antigen gene was used. A solution containing this mRNA was mixed with an ethanol-dissolved lipid solution with the composition (15) in Table 2 or an ethanol-dissolved lipid solution obtained by further adding maleimide-PEG-DMG to an ethanol-dissolved lipid solution with the composition (15) (FFT10/FFT20/DOPE/DOTAP/cholesterol/PEG-DMG/maleimide-PEG-DMG =20/20/10/10/36/2/2 (mol%)), and then further mixed with 10mM HEPES (pH7.3), and then the mixture was washed and concentrated by centrifugal ultrafiltration, so that a total of two kinds of solutions containing lipid particles exhibiting lipid compositions different from each other as lipid particles were obtained.

### · Transfection of nucleic acid into dendritic cells by lipid particles

Each of a total of two kinds of solutions containing lipid particles exhibiting lipid compositions different with each other was added to each well of the above culture plate containing dendritic cells so that the mRNA of the CAR antigen gene was 2.0 µg RNA/well. In other words, a solution containing lipid particles corresponding to each was dispensed so that lipid particles exhibiting the lipid composition (15) were contained in one well and lipid particles further containing maleimide-PEG-DMG were contained in the other well. After this dispensing, the culture plate was placed in an incubator at 37°C in a 5%CO₂ atmosphere and cultured to transfect the nucleic acid into the dendritic cells.

### · Detection of dendritic cells expressing CAR antigen

About one day after the culture, each culture plate was taken out from the incubator, cells expressing EphB4 were detected by fluorescent immunostaining, and a proportion of dendritic cells expressing the CAR antigen gene among the total number of dendritic cells was calculated.

### · Results

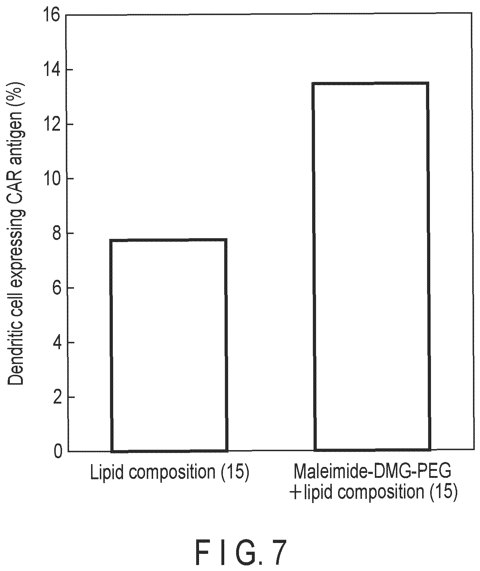

The experimental results of Example 3 are shown in FIG. 7. The numerical value on the vertical axis in FIG. 7 indicates the proportion of dendritic cells in which the CAR antigen gene is expressed, (A) on the horizontal axis indicates the case of using lipid particles with the lipid composition (15) described in Table 2, and (B) indicates the case of using lipid particles further containing the maleimide-PEG-DMG described above.

As shown in FIG. 7, it can be seen that when a gene is transferred using lipid particles further containing the maleimide-PEG-DMG described above, the proportion of CAR antigen-presenting cells is higher than that when a gene is transferred using lipid particles with the lipid composition (15) described in Table 2. In other words, it has been shown that the use of lipid particles further containing maleimide-PEG-DMG in addition to FFT10, FFT20, DOPE, DOTAP, cholesterol and PEG-DMG exhibits higher gene transfer efficiency for dendritic cells.

While certain arrangements have been described, these arrangements have been presented by way of example only, and are not intended to limit the scope of the claims. Indeed, the lipid particle described herein may be embodied in a variety of other forms; furthermore, various omissions, substitutions and changes in the form of the lipid particle described herein may be made.

The arrangements as described above include clauses below.

### Clause 1

A lipid particle (10) for producing a CAR antigen-expressing cell derived from a CD209 positive cell,
the lipid particle characterized by including a lipid membrane (1) having a lumen, and
a nucleic acid (2) containing a CAR antigen gene being contained in the lumen.

### Clause 2

The lipid particle according to Clause 1, characterized in that the lipid membrane contains 50% or more of a cationic lipid in lipid composition (molar ratio).

### Clause 3

The lipid particle according to Clause 2, characterized in that
the lipid membrane contains a lipid compound represented by the formula Q-CHR₂
wherein,
Q is a nitrogen-containing aliphatic group containing two or more tertiary nitrogen atoms and no oxygen;
Rs are each independently an aliphatic group from C₁₂ to C₂₄;
at least one R includes, in its main chain or side chain, a linking group LR selected from the group consisting of -C(=O)-O-, -O-C(=O)-, -O-C(=O)-O-, -S-C(=O)-, -C(=O)-S-, -C(=O)-S-, -C(=O)-NH-, and - NHC(=O)-.

### Clause 4

The lipid particle according to Clause 3, characterized in that the lipid compound is a lipid compound of the following formula (1-01) and/or a lipid compound of the following formula (1-02).

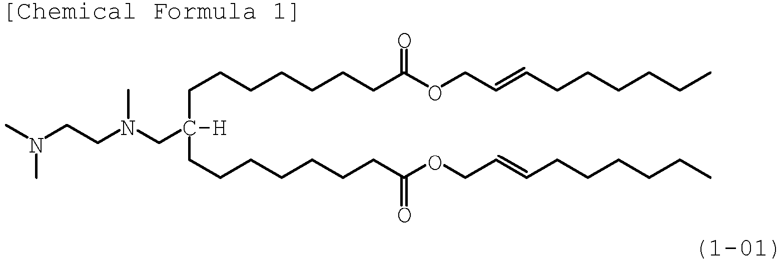

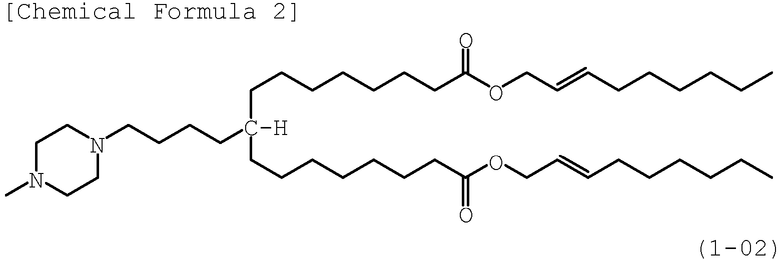

### Clause 5

The lipid particle according to Clause 4, characterized in that the lipid compound of the formula (1-01) and the lipid compound of the formula (1-02) respectively account for 10% to 30% in the lipid composition (molar ratio) of the lipid membrane, and are in equal proportions to each other.

### Clause 6

The lipid particle according to Clause 3, characterized in that the lipid membrane further contains a base lipid or a lipid that suppresses aggregation of the lipid particles.

### Clause 7

The lipid particle according to Clause 6, characterized in that the base lipid is 1,2-dioleoyl-3-trimethylammonium propane (DOTAP) and/or 1,2-dioleoyl-sn-glycero-3-phosphoethanolamine (DOPE).

### Clause 8

The lipid particle according to Clause 6, characterized in that the lipid that suppresses aggregation of the lipid particles is polyethylene glycol dimyristoyl glycerol (DMG-PEG) or maleimide DMG-PEG.

### Clause 9

The lipid particle according to Clause 4, characterized in that the lipid membrane contains the lipid compound of the formula (1-01) and the lipid compound of the formula (1-02), 1,2-dioleoyl-3-trimethylammonium propane (DOTAP) and/or 1,2-dioleoyl-sn-glycero-3-phosphoethanolamine (DOPE), cholesterol, and polyethylene glycol dimyristoyl glycerol (DMG-PEG) or maleimide DMG-PEG.

### Clause 10

The lipid particle according to Clause 1, characterized in that the CAR antigen gene is a full length or a part of any one or more genes of EphB4, EGFR variant 3, ALK and EphA2.

### Clause 11

The lipid particle according to Clause 1, characterized in that the nucleic acid is DNA, RNA, PNA, or any derivative thereof.

### Clause 12

The lipid particle according to Clause 1, characterized in that the nucleic acid is a single-stranded or double-stranded cyclic, linear or branched nucleic acid.

### Clause 13

A kit for producing a CAR antigen-expressing cell, comprising at least
the lipid particle according to any one of Clauses 1 to 12, and
a substance that improves storage stability of the lipid particle.

### Clause 14

The kit according to Clause 13, characterized in that the lipid particle is directly or indirectly attached or fixed to a solid phase.

### Clause 15

The kit according to Clause 14, characterized in that the solid phase is a container made of metal, resin, gel, or fiber, a plate, or a sheet.

### Clause 16

A method that proliferate a genetically-modified T cell (CAR-T cell) expressing a chimeric antigen receptor (CAR), the method 1, characterized by comprising:
preparing a cell population containing CAR-T cells, a CD209 positive cell, and the lipid particle according to any one of Clauses 1 to 12, respectively;
producing a CAR antigen-presenting cell by bringing the lipid particle into contact with the CD209 positive cell and transfecting the nucleic acid into the CD209 positive cell; and
co-culturing the CAR-T cell and the CAR antigen-presenting cell.

### Clause 17

The method according to Clause 16, characterized in that the CD209 positive cell is a dendritic cell.

### Clause 18

The method according to Clause 16, characterized in that the cell population containing CAR-T cells is produced from a cell population containing T cells.

### Clause 19

The method according to Clause 18, characterized in that the cell population containing T cells is peripheral blood mononuclear cells (PBMCs).

## Claims

1. A lipid particle (10) for producing a CAR antigen-expressing cell derived from a CD209 positive cell,
the lipid particle **characterized by** including a lipid membrane (1) having a lumen, and
a nucleic acid (2) containing a CAR antigen gene being contained in the lumen.

2. The lipid particle according to claim 1, **characterized in that** the lipid membrane contains 50% or more of a cationic lipid in lipid composition (molar ratio).

3. The lipid particle according to claim 2, **characterized in that**
the lipid membrane contains a lipid compound represented by the formula Q-CHR₂
wherein,
Q is a nitrogen-containing aliphatic group containing two or more tertiary nitrogen atoms and no oxygen;
Rs are each independently an aliphatic group from C₁₂ to C₂₄;
at least one R includes, in its main chain or side chain, a linking group LR selected from the group consisting of -C(=O)-O-, -O-C(=O)-, -O-C(=O)-O-, -S-C(=O)-, -C(=O)-S-, -C(=O)-S-, -C(=O)-NH-, and - NHC(=O)-.

4. The lipid particle according to claim 3, **characterized in that** the lipid compound is a lipid compound of the following formula (1-01) and/or a lipid compound of the following formula (1-02).

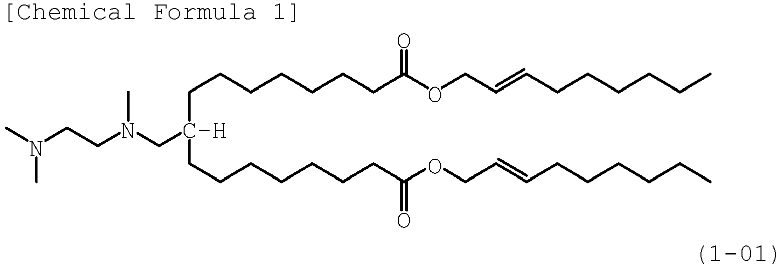

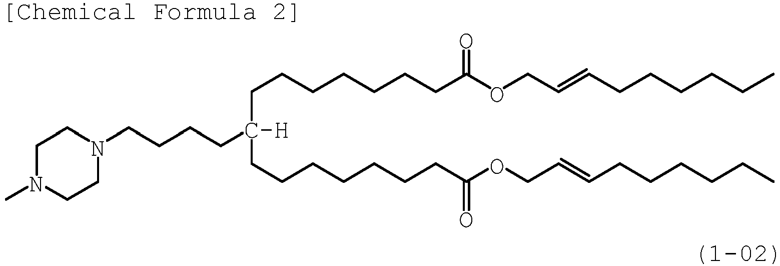

5. The lipid particle according to claim 4, **characterized in that** the lipid compound of the formula (1-01) and the lipid compound of the formula (1-02) respectively account for 10% to 30% in the lipid composition (molar ratio) of the lipid membrane, and are in equal proportions to each other.

6. The lipid particle according to claim 3, **characterized in that** the lipid membrane further contains a base lipid or a lipid that suppresses aggregation of the lipid particles.

7. The lipid particle according to claim 6, **characterized in that** the base lipid is 1,2-dioleoyl-3-trimethylammonium propane (DOTAP) and/or 1,2-dioleoyl-sn-glycero-3-phosphoethanolamine (DOPE).

8. The lipid particle according to claim 6, **characterized in that** the lipid that suppresses aggregation of the lipid particles is polyethylene glycol dimyristoyl glycerol (DMG-PEG) or maleimide DMG-PEG.

9. The lipid particle according to claim 4, **characterized in that** the lipid membrane contains the lipid compound of the formula (1-01) and the lipid compound of the formula (1-02), 1,2-dioleoyl-3-trimethylammonium propane (DOTAP) and/or 1,2-dioleoyl-sn-glycero-3-phosphoethanolamine (DOPE), cholesterol, and polyethylene glycol dimyristoyl glycerol (DMG-PEG) or maleimide DMG-PEG.

10. The lipid particle according to claim 1, **characterized in that** the CAR antigen gene is a full length or a part of any one or more genes of EphB4, EGFR, EGFR variant 3, ALK and EphA2.

11. A method that proliferate a genetically-modified T cell (CAR-T cell) expressing a chimeric antigen receptor (CAR), the method 1, **characterized by** comprising:
preparing a cell population containing CAR-T cells, a CD209 positive cell, and the lipid particle according to any one of claims 1 to 10, respectively;
producing a CAR antigen-presenting cell by bringing the lipid particle into contact with the CD209 positive cell and transfecting the nucleic acid into the CD209 positive cell; and
co-culturing the CAR-T cell and the CAR antigen-presenting cell.

12. The method according to claim 11, **characterized in that** the CD209 positive cell is a dendritic cell.

13. The method according to claim 11, **characterized in that** the cell population containing CAR-T cells is produced from a cell population containing T cells.

14. The method according to claim 13, **characterized in that** the cell population containing T cells is peripheral blood mononuclear cells (PBMCs).
